# EUROPEAN PATENT SPECIFICATION

(11) **EP 4 253 398 B1**
(45) Date of publication and mention of the grant of the patent: **05.03.2025**
(21) Application number: 23163593.9
(22) Date of filing: 22.03.2023
(51) Int. Cl.: C07K 5/12, C07K 7/64, C07K 5/117, C07K 5/107

(54) **A MECHANOCHEMICAL METHOD FOR OBTAINING OF CYCLIC PEPTIDES BY THE SOLID STATE TRANSFORMATION**
MECHANOCHEMISCHES VERFAHREN ZUR GEWINNUNG VON CYCLISCHEN PEPTIDEN DURCH FESTKÖRPERTRANSFORMATION
PROCÉDÉ MÉCANOCHIMIQUE D'OBTENTION DE PEPTIDES CYCLIQUES PAR TRANSFORMATION À L'ÉTAT SOLIDE

(30) Priority: 31.03.2022 PL 44082322
(43) Date of publication of application: 04.10.2023
(73) Proprietor: Centrum Badan Molekularnych I Makromolekularnych Pan, 90-363 Lodz (PL)
(72) Inventor: WROBLEWSKA, Aneta, Lodz (PL); WIELGUS, Ewelina, Lodz (PL); POTRZEBOWSKI, Marek, Lodz (PL)
(74) Representative: Czub, Krzysztof

(56) References cited:
- SAROJINI VIJAYALEKSHMI ET AL: "Cyclic Tetrapeptides from Nature and Design: A Review of Synthetic Methodologies, Structure, and Function", CHEMICAL REVIEWS, vol. 119, no. 17, 11 September 2019 (2019-09-11), US, pages 10318 - 10359, XP093063223, ISSN: 0009-2665, DOI: 10.1021/acs.chemrev.8b00737
- KYRIAKOS G VARNAVA ET AL: "Making Solid-Phase Peptide Synthesis Greener: A Review of the Literature", CHEMISTRY - AN ASIAN JOURNAL, WILEY-VCH, HOBOKEN, USA, vol. 14, no. 8, 20 February 2019 (2019-02-20), pages 1088 - 1097, XP072427387, ISSN: 1861-4728, DOI: 10.1002/ASIA.201801807

## Description

### The field of technology.

The subject of the invention is a method for obtaining of cyclic peptides by solid state transformation.

### State of the art.

One of the challenges of modem bioorganic chemistry is the effective synthesis of small cyclic peptides (SCPs) containing four to twelve amino acids in sequence. SCPs are characterized by a unique structures and therefore a wide spectrum of biological properties, making them an attractive target for researchers working in various fields, from medicine to materials chemistry [1]. Cyclic peptides are important to the pharmaceutical industry because these systems provide a convenient medium for binding active sites of receptors *via* non-covalent interactions *e*.*g*. hydrogen bonding, dipole-dipole or ionic contacts. Chemically modified cyclic peptides with the desired amino acid sequence are active substances that are released in the body under the influence of stimulation of a specific environment. In many cases, low toxicity and low degradability in the body compared to linear peptides are an additional advantage. Of the more than 60 peptides approved by the FDA and EMA as drugs, two-thirds are in cyclic form [2]. No wonder that many scientists in laboratories around the world are working intensively on the development of synthetic methods for obtaining cyclic peptides.

Unfortunately SCPs are difficult to synthesize. One of the reasons for these difficulties is conformational and energy constraints. In general, due to energy constraints, medium-sized (9-12-membered) rings are more difficult to form compared to smaller (4-8-membered) and larger (>12-membered) rings. This is mainly due to the fact that additional transferring interactions between substituents play a key role in the strain energy of the ring. Various synthesis strategies for the production of SCPs have recently been reviewed by Sarojini and colleagues [2]. In order to obtain any cyclic peptide in solution, the C- and N-termini of a linear peptide chain must be in close spatial proximity to facilitate intramolecular cyclization. Pseudocyclic conformation of linear peptides prefers intramolecular processes and eliminates and/or limits the possibility of intermolecular reactions. Pseudocyclic conformations are rare due to the nature of the peptide bond, which adopts mostly trans geometry, ultimately resulting in extended linear structures. The mixture of L and D amino acid configurations in the peptide chain can promote conformations favourable for cyclization. L-Proline or its enantiomer D-proline are the most common natural inducers of the appropriate spatial arrangement responsible for the "turn" of the molecule. The best results are obtained by introducing a proline in the middle of a linear peptide sequence to facilitate the reactive ends approaching space to form a new peptide bond.

In the case of solution cyclization, many factors play a role in producing the intended SCPs versus competing intermolecular reactions leading to dimer, oligomer or polymer formation. In particular, the selection of coupling reagents, the order in which they are added, and the knowledge of their interrelationships are important. Reagents can be added to the peptide solution placed in high dilution or to the reagent solution with stirring, the peptide solution can be added gradually from the syringe pump. The synthetic strategy proposed in our invention is based on a completely different philosophy, inspired by the experience of crystal engineering and based on the latest achievements in mechanochemistry.

In recent years, there has been a growing interest in applying mechanochemistry as a versatile platform for preparing various solid-state compounds [3]. Margetić and Strukil described in a comprehensive textbook a number of chemical reactions that can be carried out in a semi-solid and/or solid state using a mechanochemical approach [4]. Such transformations are possible using a variety of approaches, ranging from mortars and mechanical hand force to dedicated devices such as mixing or planetary ball mills. Research on energy transfer and grinding kinetics in this type of mill leads to the conclusion that the effective modulus of elasticity of impact media expressed by E_{eff} may be in the range of hundreds of GPa [5]. It has been proven that molecules react to an external force through bond rotation, angular bending and bond stretching [6]. Based on the observation of strained molecules, it can also be assumed that during high-energy, intensive grinding, the "topochemical principle" does not need to be treated rigorously, especially when the matter is softened by the presence of small amounts of liquid solvents (LAG). Such a strategy opens the way to obtaining "difficult" products using classical methods.

One of them, widely used, is the so-called "template method". Linear peptides are bound by coordination bonds with metal cations of appropriate size, which reduces the distance between the reacting ends of the tested systems (C- and N-terminus) to establish a peptide bond. Finally, cyclic compounds containing previously defined metals inside the peptide ring are obtained. Their removal often causes many problems; in special cases, it is only possible to carry out.

The second synthetic method of cyclic peptide systems is the "high dilution method". Linear tetrapeptides are classically cyclized in very large volume solutions. This strategy aims to eliminate the likelihood of intermolecular reactions competing with intramolecular reactions.

Both methods have limitations, and the target compounds are generally produced with low yields. An additional disadvantage (especially in the case of the second method) is the need to use a large amount of mostly organic solvents characterized by certain toxicity (chloroform, methylene chloride).

### Purpose of the invention.

The invention aims to develop an effective method for obtaining of cyclic peptides using mechanical stimuli without the need to use environmentally harmful organic solvents. The effectiveness of the method was proven using four linear peptides HCl·ProPheProPhe (HCI-PFPF) **1,** Tyr-Pro-Phe-Phe-OH (YPFF) **2** (Endomorphin-2 neuropeptide analogue), Tyr-(D)Ala-Phe-Gly (Y(D)AFG) **3** (address sequence of the neuropeptide Dermorphin) and the O-benzyl derivative Tyr-(D)Ala-Phe-Gly (Y(OBn)(D)AF) **4.** 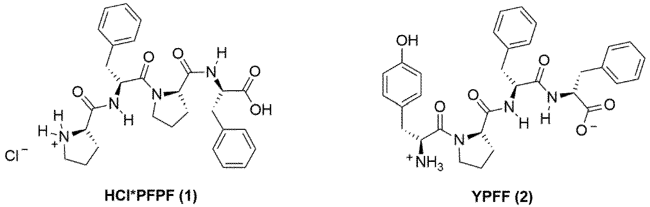 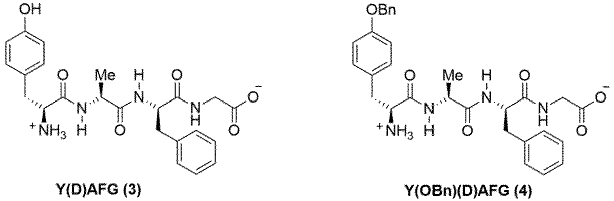 Each of the peptides used has a pseudocyclic conformation in a solid state, meeting topochemical criteria and enabling the formation of new peptide bonds and the synthesis of cyclic compounds. Mechanosynthesis carried out in ball mills using *O-*(benzotriazol-1-yl)-1,1,3,3-tetramethyluronium tetrafluoroborate (TBTU) as coupling agent and diisopropylethylamine (DIPEA) as base showed the formation of two types of cyclic products, namely tetrapeptides (CTPs) and cyclic octapeptides (COPs). The efficiency of CTP and COP is correlated with the inter- and intramolecular distances in the crystallographic unit cell between the active C and N termini involved in forming new peptide bonds. Monitoring of mechanosynthesis processes and analysis of the obtained products was based on measurements of liquid chromatography coupled with mass spectrometry (LC-MS), solid phase NMR spectroscopy (SS NMR) and liquid phase NMR.

### The summary of the invention.

The present invention is based on the unexpected observation that during grinding of solids, cyclic peptides can be obtained. The key is the pseudocyclic conformation of the peptides, in which the reactive carboxyl and amino ends are located at a distance of 4 to 10 angstroms. The cyclization method of tetrapeptides with formulas 1-4 is based on grinding three components (linear tetrapeptide, coupling reagent and base) in a ball mill for 1-3 hours. The obtained raw post-reaction mixture is washed several times with water to remove the by-products generated in the grinding process, thanks to which a faster and more accurate analysis of the obtained results is possible.

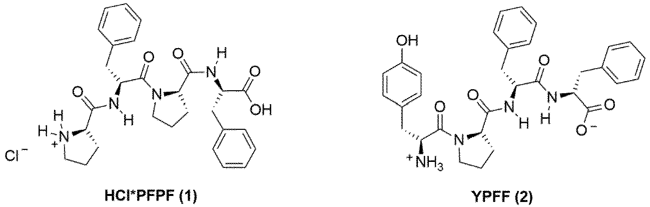

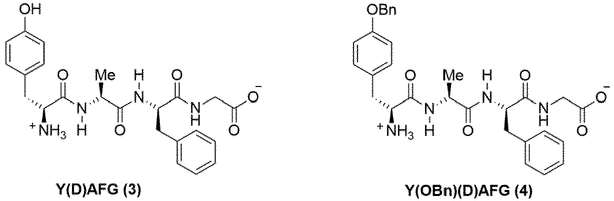

A compound from the group consisting of O-(benzotriazol-1-yl)-1,1,3,3-tetramethyluronium tetrafluoroborate, bis(dimethylamino)methylene]-1H-1,2,3-triazole 3-oxide hexafluorophosphate was selected as the coupling reagent [4,5-b]pyridinium and (1-((dimethylamino)(morpholino)methylene)-1H-benzotriazolium 3-oxide hexafluorophosphate. Diisopropylethylamine is used as the base.

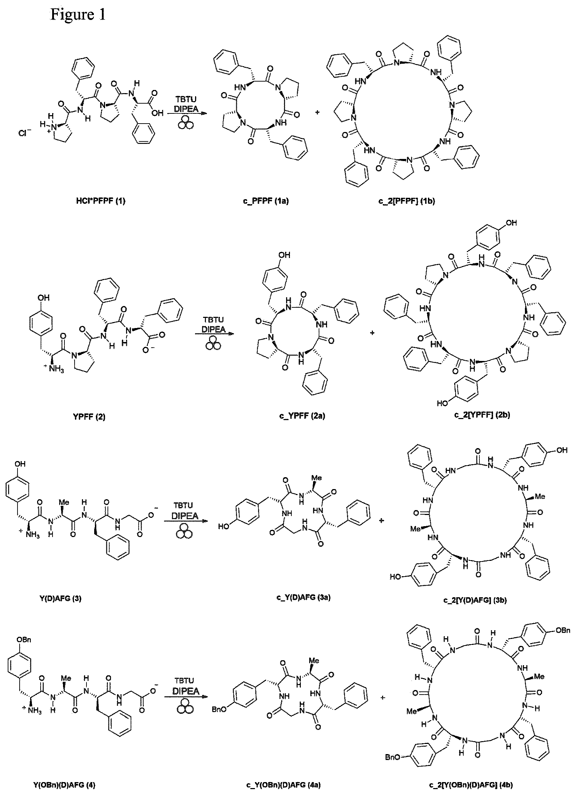

The invention may be more readily understood by referring to the accompanying drawings, in which Fig. 1 shows a general flowchart of the invention;

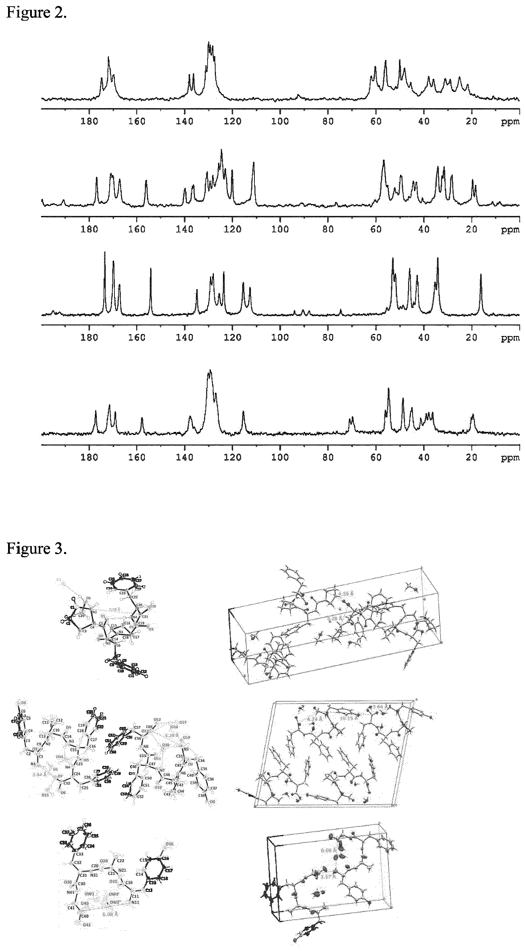

Figure 2 shows the ¹³C CP/MAS NMR spectra of linear peptide samples used for cyclization. Sample **1** is shown on the top, samples **2, 3** and **4** are displayed below. Solid state spectra were recorded at 8 kHz spinning speed.

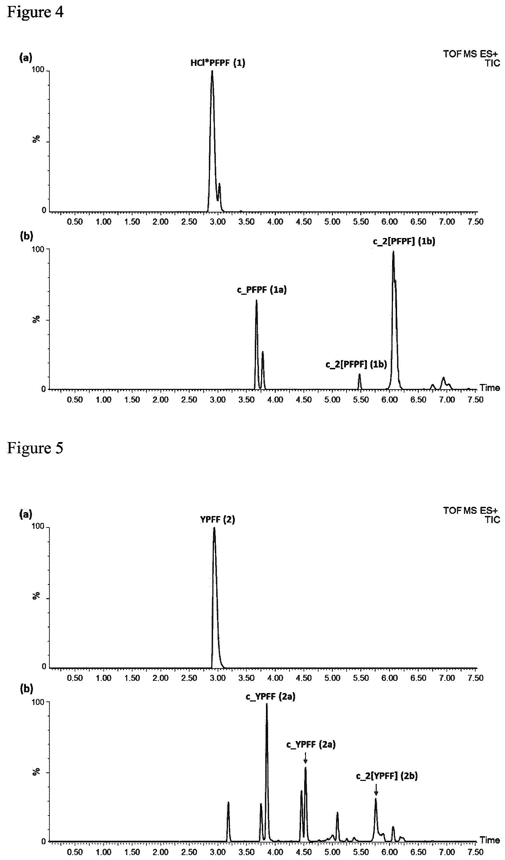

Fig. 3 shows the X-ray structures of the samples used for cyclization. Sample **1** is shown on the top, below are displayed the X-ray structures of samples **2** and **3.** Fig. 4 shows the UPLC-ESI(+)-MS chromatogram for (a) HCl·PFPF **1,** (b) triturating the sample of HCl·PFPF **1**, TBTU and DIPEA after 3 hours and washing the crude mixture with water;
Fig. 5 shows the UPLC-ESI(+)-MS chromatogram of (a) YPFF **2,** (b) triturating the sample of YPFF **2,** TBTU and DIPEA after 3 hours and washing the crude mixture with water;

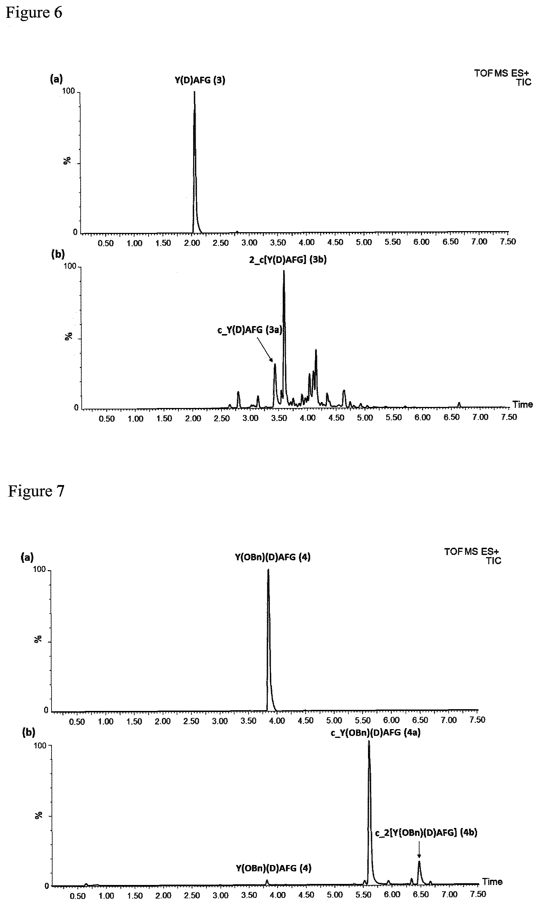

Fig 6 shows the UPLC-ESI(+)-MS chromatogram of (a) Y(D)AFG **3,** (b) triturating the sample of Y(D)AFG **3,** TBTU and DIPEA after 3 hours and washing the crude mixture with water;
Fig. 7 shows the UPLC-ESI(+)-MS chromatogram for (a) Y(OBn)(D)AFG **4,** (b) trituration of the sample Y(OBn)(D)AFG **4,** TBTU and DIPEA after 3 hours and washing the crude mixture with water;

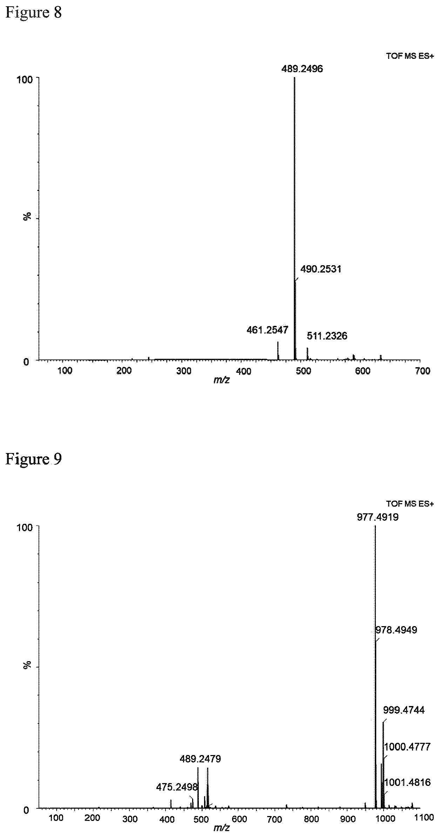

Fig. 8 shows the MS spectrum confirming the structure of the obtained cyclic tetrapeptide c_PFPF (**1a**);
Fig. 9 shows the MS spectrum confirming the structure of the obtained cyclic octapeptide c_2[PFPF] **(1b);**

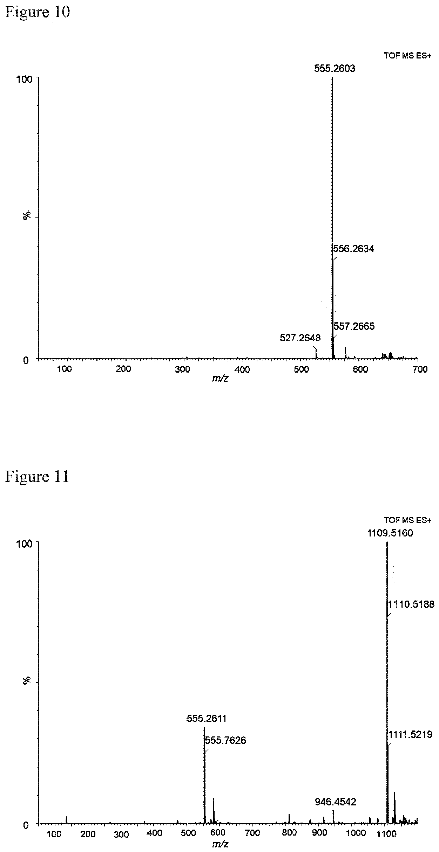

Fig. 10 shows the MS spectrum confirming the structure of the resulting cyclic tetrapeptide c_YPFF **(2a);**
Fig. 11 shows the MS spectrum confirming the structure of the obtained cyclic octapeptide c_2[YPFF] **(2b);**

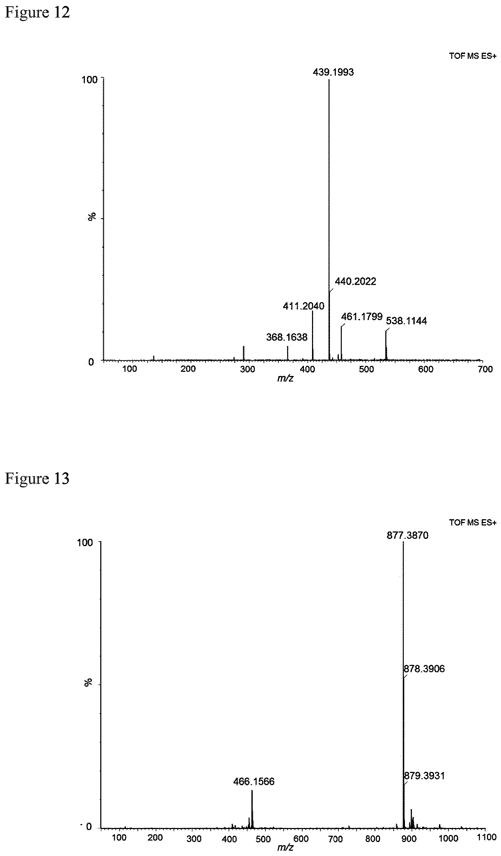

Fig. 12 shows the MS spectrum confirming the structure of the obtained cyclic tetrapeptide c_Y(D)AFG **(3a);**
Fig. 13 shows the MS spectrum confirming the structure of the obtained cyclic octapeptide c_2[Y(D)AFG] **(3b);**

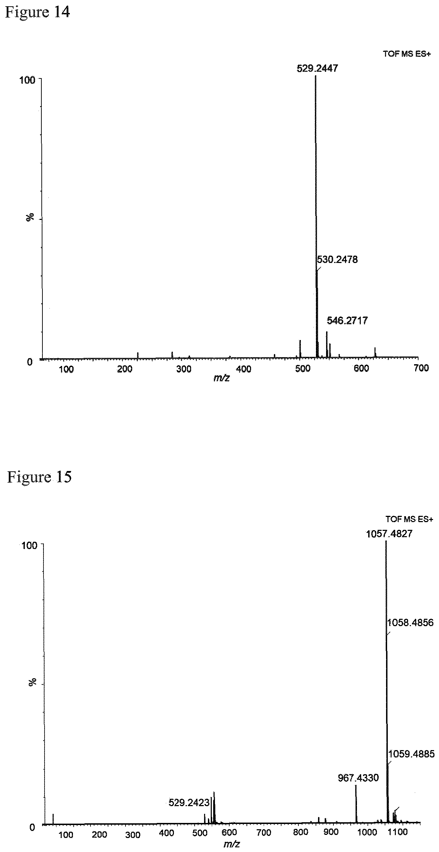

Fig. 14 shows the MS spectrum confirming the structure of the obtained cyclic tetrapeptide c_Y(OBn)(D)AFG **(4a);**
Fig. 15 shows the MS spectrum confirming the structure of the obtained cyclic octapeptide c_2[Y(OBn)(D)AFG] **(4b);**

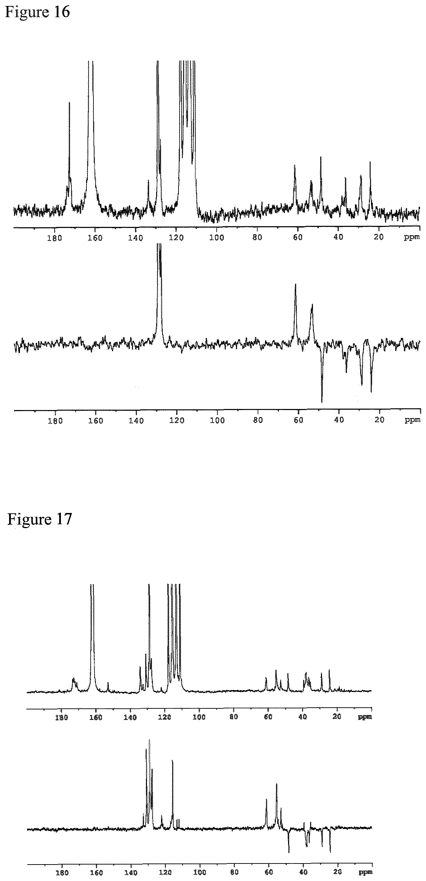

Fig. 16 shows the ¹³C NMR spectrum of cyclic peptide **1** (1a, 1b) after grinding and washing with water, recorded in deuterated trifluoroacetic acid TFA-d.
   The upper spectrum was made using the SPE (Single Pulse Experiment) basic sequence. The bottom spectrum was taken using the DEPT135 sequence.
Fig. 17 shows the ¹³C NMR spectrum of cyclic peptide **2** (2a, 2b) after grinding and washing with water, recorded in deuterated trifluoroacetic acid TFA-d. The upper spectrum used the SPE (Single Pulse Experiment) basic sequence. The lower spectrum was made using the DEPT135 sequence.

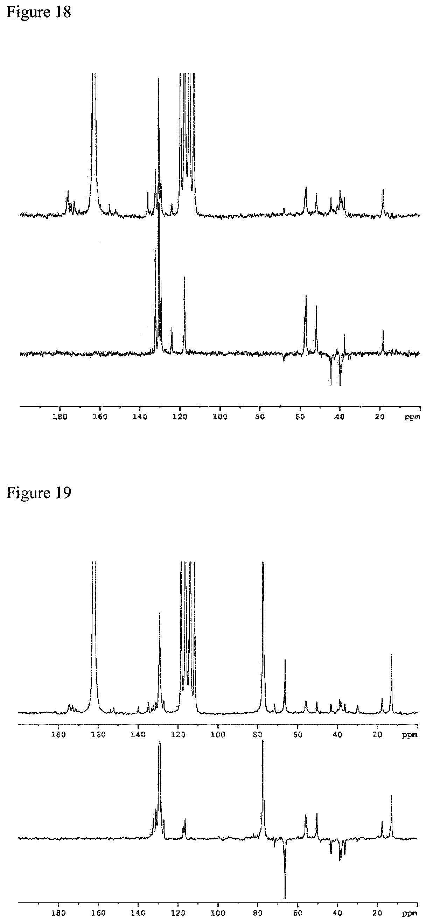

Fig. 18 shows the ¹³C NMR spectrum of cyclic peptide **3** (3a, 3b), after grinding and washing with water, recorded in deuterated trifluoroacetic acid TFA-*d*. The upper spectrum was made using the SPE (Single Pulse Experiment) basic sequence. The bottom spectrum was taken using the DEPT135 sequence.
Fig. 19 shows the ¹³C NMR spectrum of cyclic peptide **4** (4a, 4b), after grinding and washing with water, recorded in deuterated trifluoroacetic acid TFA-*d*. The upper spectrum was made using the SPE (Single Pulse Experiment) basic sequence. The lower spectrum was made using the DEPT135 sequence.

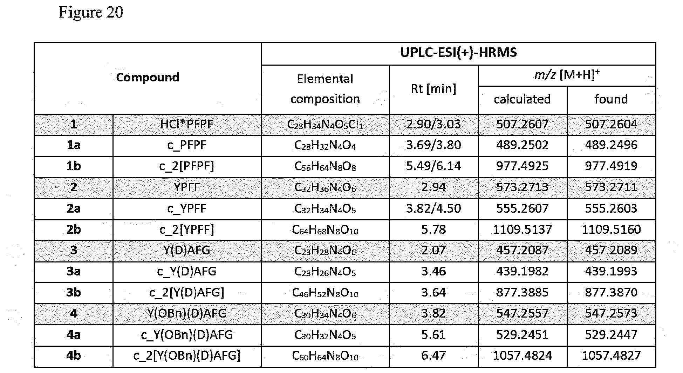

Fig. 20 is a table showing UPLC-ESI(+)-MS data; UPLC retention time (Rt, min) and *m*/*z* for [M+H]⁺ ions in the positive ionization mode of substrates and cyclic products (tetra- and octapeptides);

The invention is explained in more detail by means of non-limiting examples:

### Example 1

50 mg of linear tetrapeptide hydrochloride Pro-Phe-Pro-Phe **1,** 59 mg of TBTU coupling reagent or 70 mg of HATU or 77 mg of HDMB, and 162 µl of DIPEA base, one 10 mm diameter stainless steel grinding ball placed in a stainless steel grinding vessel with a volume of 10 mL and mixed in an MM200 ball mill at a frequency of 25 Hz for 1 hour. The crude reaction mixture was washed 5 times with water (5 x 10 mL), and the solvent was allowed to evaporate freely. The yield of the cyclization process leading to the cyclic tetrapeptide **1a** and the cyclic octapeptide **1b** is 62%. The structures of the compounds are confirmed by the MS and NMR spectra recorded in deuterated trifluoroacetic acid (TFA-*d*).

### Example 2

50 mg of Tyr-Pro-Phe-Phe-OH **2** linear tetrapeptide, 70 mg of TBTU coupling reagent or 82 mg of HATU or 92 mg of HDMB and 153 µl of DIPEA base, one 10 mm diameter stainless steel grinding ball placed in a stainless steel grinding vessel 10 mL volume of stainless steel and mixed in an MM200 ball mill at 25 Hz for 3 hours. The crude reaction mixture was washed 5 times with water (5 x 10 mL). The cyclization process yielding the cyclic tetrapeptide **2a** and the cyclic octapeptide **2b** is 47%. The structures of the compounds are confirmed by the MS and NMR spectra recorded in deuterated trifluoroacetic acid (TFA-*d*).

### Example 3

50 mg of Tyr-(D)Ala-Phe-Gly **3** linear tetrapeptide, 88 mg of TBTU coupling reagent or 104 mg of HATU or 115 mg of HDMB, and 193 µl of DIPEA base, one 10 mm diameter stainless steel grinding ball was placed in a 10 mL of stainless steel and mixed in an MM200 ball mill at 25 Hz for 3 hours. The crude reaction mixture was washed 5 times with water (5 x 10 mL). The yield of the cyclization process leading to the cyclic tetrapeptide **3a** and the cyclic octapeptide **3b** is 79%. The structures of the compounds are confirmed by the MS and NMR spectra recorded in deuterated trifluoroacetic acid (TFA-*d*).

### Example 4

50 mg linear tetrapeptide Tyr(OBn)-(D)Ala-Phe-Gly **4,** 73 mg TBTU coupling reagent or 86 mg HATU or 96 mg HDMB and 161 µl DIPEA base, one 10 mL stainless steel grinding ball and mixed in the MM200 ball mill at 25 Hz for 3 hours. The crude reaction mixture was washed 5 times with water (5 x 10 mL). The cyclization process yielding the cyclic tetrapeptide **4a** and the cyclic octapeptide **4b** is 67%. We have yet to confirm this. The structures of the compounds are confirmed by the MS and NMR spectra recorded in deuterated trifluoroacetic acid (TFA-*d*).

### Advantages of the invention.

The mechanochemical method of preparation described in the invention has many advantages. Processes taking place in this way usually run clean, effectively and efficiently [7]. An additional advantage is the elimination of disadvantages that appear in the classic synthetic approach, such as the purification and recycling of solvents. Wet procedures are not only energy-intensive, but also problematic for the environment. Therefore, syntheses carried out in condensed matter are justified in economic and ecological terms [8].

### Literature:

[1] Wang, X.; Lin, M.; Xu, D.; Lai, D.; Zhou, L. Structural Diversity and Biological Activities of Fungal Cyclic Peptides, Excluding Cyclodipeptides. Molecules 22, 2069, doi:10.3390/molecules22122069 (2017).
[2] a) Zorzi, A.; Deyle, K.; Heinis, C. Cyclic peptide therapeutics: past, present and future. Curr. Opin. Chem. Biol. 38, 24-29 doi:10.1016/j.cbpa.2017.02.006 (2017); b) Zhang, H.; Chen, S. Cyclic peptide drugs approved in the last two decades (2001-2021). RSC Chem. Biol. 3, 18-31, doi: 10.1039/d1cb00154j (2022).
[3] Sarojini, V.; Cameron, A.J.; Varnava, K.G.; Denny, W.A.; Sanjayan, G. Cyclic Tetrapeptides from Nature and Design: A Review of Synthetic Methodologies, Structure, and Function. Chem. Rev. 119, 10318-10359, doi:10.1021/acs.chemrev.8b00737 (2019).
[4] James, S.L.; Adams, Ch.J.; Bolm, C.; Braga, D.; Collier, P.; Friscic, T.; Grepioni, F.; Harris, K.D.M.; Hyett, G.; Jones, W.; Krebs, A.; Mack, J.; Maini, L.; Orpen, A.G.; Parkin, I.P.; Shearouse, W.C.; Steedk, J.W.; Waddel, D.C. Mechanochemistry: opportunities for new and cleaner synthesis. Chem. Soc. Rev. 41, 413-447, doi:10.1039/C1CS15171A (2012).
[5] Margetić, D. & Strukil, V. *Mechanochemical Organic Synthesis.* 1st edn, (Elsevier, 2016).
[6] Chen, Z.; Lu, S.; Mao, Q.; Buekens, A.; Wang, Y.; Yan, J. Energy transfer and kinetics in mechanochemistry. Environ. Sci. Pollut. Res. Int. 24, 24562-24571, doi:10.1007/s11356-017-0028-9 (2017).
[7] Bo, G.D. Mechanochemistry of the mechanical bond. Chem. Sci. 9, 15-21, doi:10.1039/C7SC04200K (2018).
[8] Tan, D.; Garcia, F. Main group mechanochemistry: from curiosity to established protocols. Chem. Soc. Rev. 48, 2274-2292, doi:10.1039/C7CS00813A (2019).
[9] Baig, R.B.N.; Varma, R.S. Alternative energy input: mechanochemical, microwave and ultrasound-assisted organic synthesis. Chem. Soc. Rev. 41, 1159-1584, doi:10.1039/C1CS15204A (2012).

## Claims

1. A mechanochemical method for obtaining of cyclic peptides, **characterized in that** it is linear tetrapeptides with a forced pseudocyclic conformation with closely located active reactive carboxyl and amino termini of the formulas **1-4** is subjected to grinding in the presence of a coupling reagent and a base for 1 to 3 hours, after which the ground raw post-reaction mixture is washed several times with water.

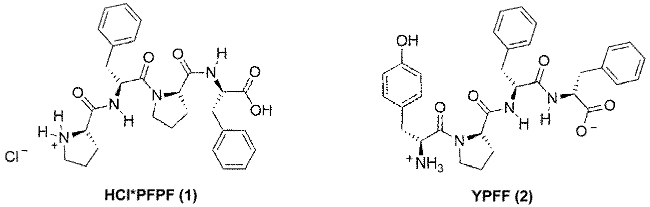

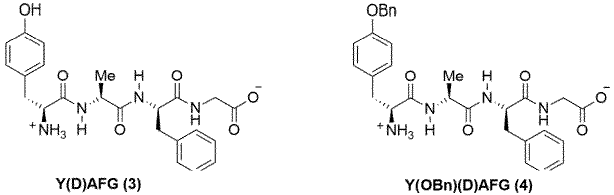

2. The method according to claim 1, wherein the tetrapeptides' reactive carboxyl and amino termini are 4 to 10 angstroms apart.

3. The method according to claim 1, **characterized in that** a compound from the group consisting of *O*-(benzotriazol-1-yl)-1,1,3,3-tetramethyluronium tetrafluoroborate, bis(dimethylamino)methylene]-3-oxide hexafluoro-phosphate-1*H-*1,2,3-triazolo[4,5-b]pyridinium and (1-((dimethylamino)-(morpholino)methylene)-1*H*-benzotriazole-3-oxide hexafluorophosphate is selected as the coupling reagent.

4. The method according to claim 1, wherein the base is diisopropylethylamine.

5. The method according to claim 1, is **characterized in that** the grinding is carried out in a ball mill.

## Patentansprüche

1. Ein mechanochemisches Verfahren zur Gewinnung von cyclischen Peptiden zeichnet sich dadurch aus, dass lineare Tetrapeptide mit Formeln von 1 bis 4 und mit erzwungener pseudozyklischer Konformation mit eng beieinander liegenden aktiven und reaktiven Carboxyl- und Aminogruppen werden sie in Gegenwart von Kupplungsreagenz und Base für 1 bis 3 Stunden gemahlen, wonach das gemahlene rohe Nachreaktionsgemisch nach der Reaktion mehrmals mit Wasser gewaschen wird.

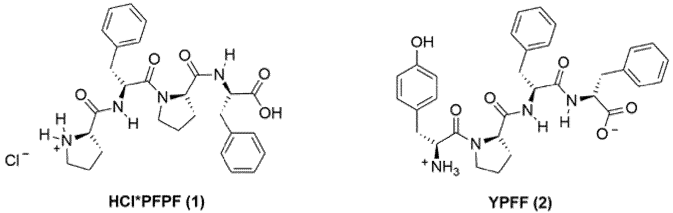

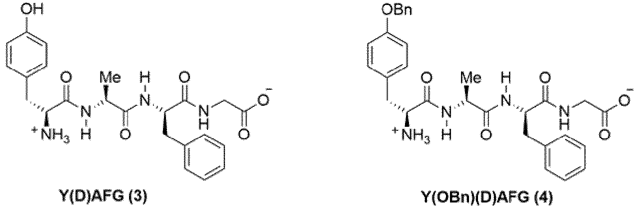

2. Das in Nummer 1 vorbehaltene Verfahren ist **dadurch gekennzeichnet, dass** die reaktiven Carboxyl- und Aminogruppen der Tetrapeptide durch 4 bis 10 Ångström voneinander getrennt sind.

3. Das in Anspruch 1 beschriebene Verfahren zeichnet sich dadurch aus, dass als Kupplungsreagenz eine Verbindung ausgewählt wird, die aus Tetrafluorborat O-(benzotriazol-1-yl)-1,3-yl)-1,3-triazol[4,5-b]pyridin und (1-(((dimethylamino)-(morpholin)methylen)-1H-benzotriazol-3-hexafluorphosphatoxid besteht.

4. Verfahren nach Anspruch 1, wobei die Base Diisopropylethylamin ist.

5. Ein Verfahren nach Anspruch 1 ist **dadurch gekennzeichnet, dass** das Mahlen in einer Kugelmühle durchgeführt wird.

## Revendications

1. Un procédé mécanochimique d'obtention de peptides cycliques est **caractérisé par le fait que** les tétrapeptides linéaires présentés par les formules 1 à 4, avec une conformation pseudocyclique forcée et contenant des groupes terminaux réactifs, sont broyés en présence d'un réactif de couplage et d'une base pendant 1 à 3 heures, après quoi le mélange de brut broyé après réaction est lavé plusieurs fois à l'eau.

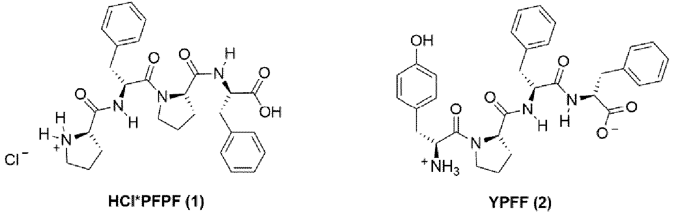

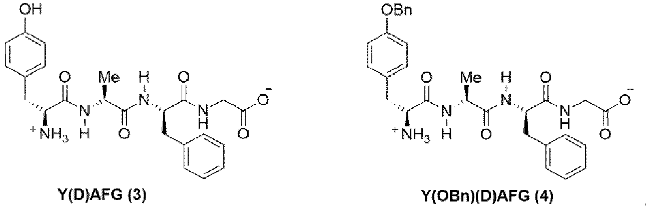

2. La méthode se **caractérise par le fait que** les groupes terminaux amino et carboxyle des tétrapeptides cyclisés sont espacés de 4 à 10 angströms.

3. Le procédé décrit selon la revendication 1 est **caractérisé par** le choix d'un composé contenant O-(benzotriazol-1-yl)-1,1,3,3-tétraméthylronium, bis(diméthylamino)méthylène]-3-hexafluorophosphate-tétrafluoromorphane-1H-1,2,3-triazole[4,5-b]pyridine et (1-(diméthylamino)-(morpholine)méthylène)-1H-benzotriazol-3-hexafluorophosphate comme réactif de couplage.

4. La méthode réservée au point 1 est **caractérisée par le fait que** la base est la diisopropyléthylamine.

5. Le procédé selon la revendication 1 est **caractérisé par le fait que** le broyage a lieu dans un broyeur à boulets.
